# EUROPÄISCHE PATENTSCHRIFT

(11) **EP 2 745 861 B1**
(45) Veröffentlichungstag und Bekanntmachung des Hinweises auf die Patenterteilung: **14.09.2016**
(21) Anmeldenummer: 13198664.8
(22) Anmeldetag: 19.12.2013
(51) Int. Cl.: A61M 1/16, A61L 2/00, A61L 2/04

(54) **Blutreinigungsmaschine mit beheiztem Flüssigkeitskreislauf**
Blood cleaning machine with heated fluid circuit
Machine de nettoyage du sang avec circuit de liquide chauffé

(30) Priorität: 24.12.2012 DE 102012113086
(43) Veröffentlichungstag der Anmeldung: 25.06.2014
(73) Patentinhaber: B. Braun Avitum AG, 34212 Melsungen (DE)
(72) Erfinder: Ritter, Kai-Uwe, 91126 Rednitzhembach (DE); Heinemann, Thore, 34320 Söhrewald (DE); Niemetz, Günter, 34212 Melsungen (DE)
(74) Vertreter: Winter, Brandl, Fürniss, Hübner, Röss, Kaiser, Polte - Partnerschaft mbB

(56) Entgegenhaltungen:
- WO-A1-96/40314
- FR-A5- 2 120 507
- US-A- 3 228 465
- US-A- 3 738 382
- US-A- 5 948 247

## Beschreibung

Die vorliegende Erfindung betrifft eine Blutreinigungsmaschine mit einem mittels einer Heizeinrichtung temperierbaren maschineninternen Flüssigkeitskreislauf gemäß dem Oberbegriff des Anspruchs 1.

### Hintergrund der Erfindung

Blutreinigungsmaschinen dienen in der Regel zur Anwendung eines extrakorporalen Blutreinigungsersatzverfahrens (beispielsweise Dialyse) insbesondere im Fall eines Nierenversagens. Dabei ist ein solches Blutreinigungsersatzverfahren oder Dialyse neben der Nierentransplantation die wichtigste Nierenersatztherapie bei chronischem Nierenversagen und eine der Behandlungsmöglichkeiten bei akutem Nierenversagen.

Unter dem Begriff "Dialyse" wird hierbei ein Stoffaustausch über eine in einem Dialysator angeordnete Membran verstanden, wobei auf der einen Membranseite Blut/Plasma und auf der anderen Seite der Membran die Reinigungsflüssigkeit oder auch Dialysierflüssigkeit anliegt. Im Kornkreten wird Blut aus einem Patienten in einem extrakoporalen Blutkreislauf zum Dialystor der Maschine geführt und dort an der Membran vorbeigeleitet. Auf der Maschinenseite wird vorzugesweise Wasser, das für eine Dialyse geeignet ist, aus einem Speicher oder einer externen Leitung entnommen, in einem maschineninternen Fluid- oder Flüssigkeitskreis zu einer Dialysierflüssigkeit aufbereitet und die frische Dialysierfüssigkeit ebenfalls zum Dialysator geführt um dort Schadstoffe aus dem vorbeiströmenden Blut aufzunehmen.

Bei der sogenannten Hämodialyse wird hierbei nach dem Prinzip des Konzentrationsausgleichs kleinmolekularer Substanzen zweier Flüssigkeiten verfahren, die durch eine semipermeable Membran im Dialysator getrennt sind (Osmose). Dabei befindet sich von der Filtermembran getrennt auf der einen Membranseite das aus dem Patientenkörper abgeführte Blut mit Nephrotoxinen, Elektrolyten (insbesondere Kalium und Phosphat) sowie harnpflichtige Substanzen. Auf der anderen Membranseite befindet sich die keimarme, ggf. aufbereitete Dialyselösung (Dialysierflüssigkeit), deren Wasser im Bedarfsfall beispielsweise bei einer Online - Zubereitung durch Umkehrosmose oder anderen bekannten Maßnahmen aufbereitet wurde, die zunächst frei von Abfallprodukten ist und die einen an den jeweiligen Bedürfnissen des Patienten orientierten Anteil an Elektrolyten aufweist.

Die semipermeable Filtermembran (Dialysatormembran) zwischen Blut und Dialysierflüssigkeit besitz notwendigerweise Poren, die kleine Moleküle wie Wasser, Elektrolyte und harnpflichtige Substanzen (z.B. Harnstoff, Harnsäure) durchlassen, aber große Moleküle wie Eiweiße und Blutzellen zurückhalten.

Bevor die Dialysierflüssigkeit bzw. Reinigungsflüssigkeit im Dialysator am Blut des Patienten vorbei strömt, muss diese von der ursprünglichen Eintrittstemperatur von typischer Weise ca. 10°C auf eine höhere Temperatur, in etwa in der Höhe der Bluttemperatur, aufgeheizt werden (ca. 37°C), um ein Auskühlen des Patienten zu vermeiden. Nachdem die Dialysierflüssigkeit durch den Dialysator geströmt ist, um dabei Schadstoffe aus dem Blut aufzunehmen, gilt sie als verbraucht und gelangt in einen Abfluss zur späteren Entsorgung.

### Stand der Technik

Aus dem Stand der Technik ist es bekannt, einen Wärmetauscher einzusetzen, um die thermische Energie der verbrauchten Dialysierflüssigkeit für ein Vorheizen von frischer (kalter) Reinigungsflüssigkeit bzw. von Dialyse - geeignetem Wasser zu nutzen bzw. teilweise rückzugewinnen und somit den Energieverbrauch der Dialysemaschine, verursacht durch das Aufheizen der Dialysierflüssigkeit bzw. des Dialyse - geeigneten Wassers, zu verringern. In diesem Fall wird die (noch kalte) Dialysierflüssigkeit oder das Wasser vor/mit Eintritt in den Flüssigkeitskreislauf über den Wärmetauscher von der verbrauchten sowie zum Abfluss geleiteten Dialysierflüssigkeit vorerwärmt.

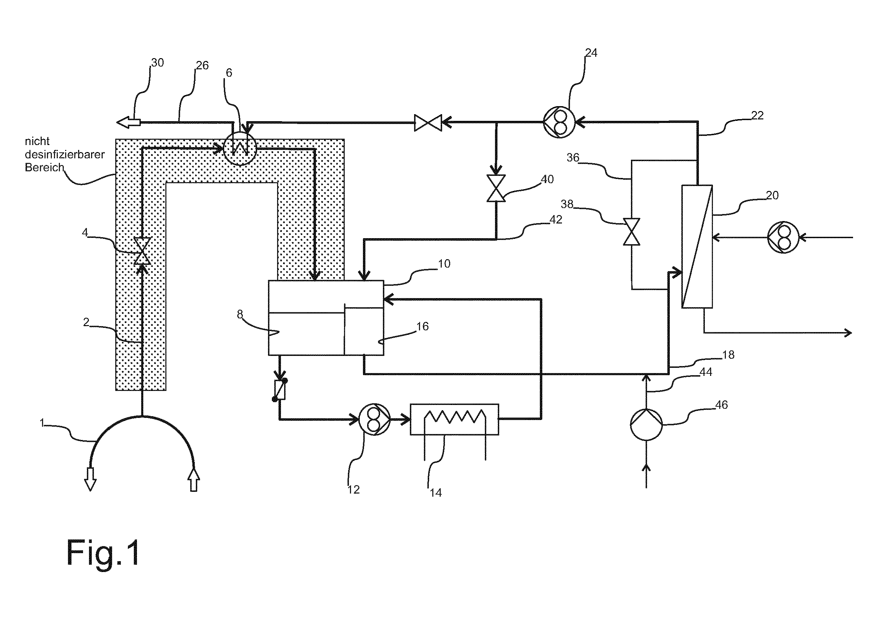

In der Fig. 1 ist ein (maschineninterner) Flüssigkeitskreislauf einer Blutreinigungsmaschine (Dialysemaschine) prinzipiell dargestellt, wie er auch aus dem Stand der Technik bekannt ist.

Demzufolge bildet der Flüssigkeitskreislauf grundsätzlich zwei schaltbare Kreisläufe, nämlich einen Reinigungs- oder Betriebskreislauf und einen Desinfektionskreislauf, bzw. der Flüssigkeitskreislauf ist in zwei Funktionsmodi, nämlich dem Blutreinigungsmodus (Betriebsmodus) und dem Desinfektionsmodus betreibbar.

Im Konkreten ist der Flüssigkeitskreislauf gemäß dem Stand der Technik über eine Zuführleitung mit darin angeordnetem Zulaufventil an eine sogenannte Osmose - Ringleitung oder einfach Wasserleitung angeschlossen, aus der Flüssigkeitsverluste während einer Blutreinigungsbehandlung in ausgewählter Weise ausgeglichen werden können. Die Zuführleitung des zur Dialyse geeigneten Wassers führt zunächst in einen Wärmetauscher und gelangt von dort in vorgewärmtem Zustand (etwa 25°C) in einen ersten Tank einer Wasser - Aufbereitungsanlage. Von dort wird das vorgewärmte und entgaste Wasser über eine Saugpumpe in eine vorzugsweise elektrisch betriebene Heizeinrichtung gepumpt, in der das Wasser auf eine Temperatur von ca. 37°C aufgeheizt wird, um dann in einen zweiten Tank der Wasser - Aufbereitungsanlage weitergeleitet zu werden, der in den Reinigungs- oder Betriebskreislauf integriert ist.

Wie aus der Fig. 1 zu entnehmen ist, führt eine erste Förderleitung das aufgeheizte sowie nunmehr zu einer Reinigungsflüssigkeit aufbereitete Wasser zu einem Dialysator, in welchem die Flüssigkeit an einer Membran vorbeiströmt, welche die Reinigungsflüssigkeit von dem Patientenblut trennt. Nachdem die Reinigungsflüssigkeit mit zu entfernenden Substanzen aus dem Patientenblut kontaminiert ist, wird die verbrauchte Flüssigkeit über eine dem Dialysator nachgeschaltete zweite Förderleitung mit darin angeschlossener Flusspumpe und einem daran sich anschließenden Sperrventil bzw. Abflussventil in den Wärmetauscher geleitet, um dort die in der Dialysierflüssigkeit gespeicherte Wärme (immer noch ca. 35°C) an das neu zugeführte Wasser abgeben zu können, um dieses vorzuwärmen. Anschließend wird die weitgehend abgekühlte Dialysierflüssigkeit über einen dem Wärmetauscher nachgeschalteten Abfluss entsorgt.

Wie vorstehend bereits angedeutet wurde, soll die in den Dialysator eingeleitete Dialysierflüssigkeit möglichst keimarm sein. Zu diesem Zweck ist an die erste Förderleitung ein Desinfektionsmittelleitungszweig angeschlossen, aus dem über eine Desinfektionspumpe wahlweise ein Desinfektionsmittel in die erste Förderleitung einleitbar ist.

Gemäß der Fig. 1 ist ein Dialysator - Umgehungskanal (Bypass) vorgesehen, der die erste und zweite Förderleitung wahlweise kurzschließt, wofür im Umgehungskanal ein Sperrventil bzw. Umgehungsventil für ein wahlweises Öffnen und Schließen des Umgehungskanals angeordnet ist. Darüber hinaus ist zwischen der Flusspumpe und dem Abflussventil ein Zirkulationsleitungszweig angeschlossen, in der ein wahlweise zu öffnendes / schließendes Sperrventil bzw. Zirkulationsventil angeordnet ist und welche in den zweiten Tank der Wasser - Aufbereitungsanlage einmündet.

Im normalen Dialysebetriebsmodus sind das Zirkulationsventil und das Umgehungsventil geschlossen, wohingegen das Abflussventil und das Zulaufventil geöffnet sind. Die Saugpumpe sowie die Flusspumpe sind in Betrieb, wohingegen die Desinfektionspumpe abgeschaltet ist und dabei gleichzeitig ein Einströmen von Reinigungsflüssigkeit in die Desinfektionsleitung verhindert. In dieser Betriebsstellung wird die Reinigungsflüssigkeit in einem offenen Kreis über den Wärmetauscher und die nachfolgende Heizeinrichtung auf Betriebstemperatur gebracht und dan durch den Dialysator gepumpt. Die verbrauchte Dialysierflüssigkeit wird zurück in den Wärmetausche geführt, um dort die frische, unverbrauchte Dialysierflüssigkeit vorzuwärmen und wird anschließend über den Ablauf entsorgt.

Nach dem Stand der Technik wird zum Desinfizieren des Flüssigkeitskreislaufs unter anderem eine sogenannte Heißdesinfektion verwendet. Dazu wird ein Wasser - Desinfektionsmittel - Gemisch im Flüssigkeitskreislauf auf > 85°C aufgeheizt und zirkuliert dort für eine bestimmte Zeit.

Im Konkreten werden hierfür das Zulaufventil und das Abflussventil geschlossen, wohingegen das Umgehungsventil und das Zirkulationsventil geöffnet werden. Des Weiteren wird die Saugpumpe abgeschaltet und statt dessen die Desinfektionspumpe in Betrieb gesetzt. In dieser Betriebsstellung zirkuliert das Desinfektionsmittel durch die erste und zweite Förderleitung über den Umgehungskanal sowie über die Zirkulationsleitung und den zweiten Tank zurück in die erste Förderleitung.

Wie aus der vorstehenden Beschreibung zu entnehmen ist, ist der Wärmetauscher vom Desinfektionskreislauf ausgeschlossen. In anderen Worten ausgedrückt, gelangt das Desinfektionsmittel, wenn überhaupt, so doch nur erst am Ende des Desinfektionszyklus lediglich in die eine (Abfluss-) Seite des Wärmetauschers, nämlich dann, wenn das Zirkulationsventil geschlossen und dafür das Abflussventil für ein Entsorgen des Desinfektionsmittels geöffnet wird. Die gegenüberliegende Wasser - Zulaufseite des Wärmetauschers wird überhaupt nicht vom Desinfektionsmittel erreicht. Darüber hinaus durchströmt das abfließende Desinfektionsmittel vergleichsweise schnell den Wärmetauscher und kann somit seine Desinfektionswirkung nicht voll entfalten.

Um dennoch eine gewisse Reinigung des Wärmetauschers herbeizuführen, wird dieser auf der Wasser - Zulaufseite mittels entnommenem Wasser vor der Behandlung eines Patienten für einige Minuten durchgespült, um zumindest eine gewisse Keimreduktion zu bewirken. Jedoch entspricht diese durch bloßes Spülen erreichte Keimreduktion sicherlich nicht einer adäquaten Desinfektion, sodass nach wie vor ein Restrisiko an einer kontaminierten Dialysierflüssigkeit verbleibt.

Nach Ablauf der vorgegebenen Desinfektionszeit muss des Weiteren das Desinfektionsmittel möglichst schnell ausgespült und die Maschine möglichst schnell wieder auf ca. 35°C abgekühlt werden, um die nächste Behandlungsphase einleiten zu können. Da jedoch das immer noch heiße Desinfektionsmittel durch den Wärmetauscher auf dessen Ausguss - Seite geführt wird und dabei gleichzeitig das einströmende Wasser auf der Wasser - Zulaufseite des Wärmetauschers erwärmt, wird folglich erwärmtes Wasser zum Durchspülen des Wärmetauschers und zum Kühlen des überhitzten Flüssigkeitskreislaufs verwendet, wodurch sich die Abkühlung der Maschine verzögert.

Zusammenfassend lassen sich die im Stand der Technik ergebenden Probleme wie Folgt darstellen:
- Der Eingangsbereich der Dialysemaschine kann konstruktiv bedingt nicht desinfiziert werden. Deshalb wird dieser Bereich bei der Konstruktion einer Dialysemaschine mit möglichst geringen Oberflächen und kurzen Schlauchlängen ausgeführt. Es besteht bei jeder Oberfläche dennoch die Möglichkeit, dass sich Mikroorganismen anlagern und einen sogenannten Biofilm ausbilden. Ein hocheffizienter Wärmetauscher würde daher die Kontaktfläche um ein mehrfaches gegenüber einer Ausführung ohne Wärmetauscher vergrößern. Eine solche Fläche undesinfiziert zu lassen, wäre daher nicht akzeptabel.
- Ein Zuschalten der Wasserzulaufstrecke in den Desinfektionskreislauf ist mit dem Risiko behaftet, dass bei einem Unterdruck in der Wasseraufbereitungsanlage das mit Desinfektionsmittel versetzte Wasser in den externen Wasserkreislauf des Dialysezentrums gelangt und dort zu einer Schädigung der Patienten führt. Um eine solche Lösung dennoch umzusetzen, wären aufwändige Vorrichtungen nötig, um sicher ein Rückströmen beispielsweise in die Wasseraufbereitungsanlage zu verhindern.
- Die Diaysierflüssigkeit muss vom Zulauf, beispielsweise mit 10°C auf etwa 37°C erwärmt werden. Für eine Behandlung werden ca. 120l an Flüssigkeit benötigt, was zu einem Wärmeenergiebedarf von etwa 3,6 kWh führt. Wenn kein Wärmetauscher vorhanden ist, wird diese Heizleistung elektrisch aufgebracht, wodurch der Energiebedarf der Dialysemaschine ansteigt.
- Bei den Dialysemaschinen gemäß dem Stand der Technik wird in der Regel ein Wärmetauscher eingesetzt, jedoch mit vergleichsweise kleiner Oberfläche, um eine geringere Verkeimung zu verursachen. Daher kann nur ein kleiner Teil der eingesetzten Energie wiederverwendet werden. Typische Werte für eingesetzte Wärmetauscher liegen bei weit < 50% Wirkungsgrad, sodass rechnerisch immer noch ein Mindestbedarf von 1,8 kWh besteht.
- Bei den vorhandenen Wärmetauschern treten beim Abkühlen der Maschine nach Desinfektionszyklen Verzögerungen auf, da der Wärmetausche auch beim finalen Spülprozess in der Zuführleitung zwischengeschaltet ist. Während der Ausspülphase, am Ende der Desinfektion, soll die Maschine möglichst schnell wieder eine Temperatur < 40°C erreichen, um möglichst schnell mit der nächsten Behandlung beginnen zu können. Die Heißdesinfektion wird jedoch mit einer Temperatur > 85°C durchgeführt. Beim Stand der Technik würde beim Ausspülen das zulaufende Wasser jedoch unerwünscht durch den permanent zwischengeschalteten Wärmetauscher aufgeheizt, was die Ausspül- und damit Abkühlphase verlängert.

Darüber hinaus ist beispielsweise aus der WO 96/40314 A1 eine desinfizierbare Maschine zur extrakorporalen Blutbehandlung gemäß dem Oberbegriff des Anspruchs 1 bekannt. Weitere desinfizierbare Maschinen zur extrakorporalen Blutbehandlung mit einem maschinenseitigen Fluidkreislauf sowie mit einem Wärmetauscher sind aus der FR 2 120 507 A5 und der US 5 948 247 A bekannt. Ein Wärmetauscher, der sich in einer Maschine zur extrakorporalen Blutbehandlung einsetzen lässt, ist beispielsweise in der US 3 228 465 A beschrieben. Zudem ist beispielsweise aus der US 3 738 382 A eine desinfizierbare Maschine zur Herstellung einer Dialysierflüssigkeit bekannt.

### Kurzfassung der Erfindung

In Anbetracht der vorstehend erläuterten Problematik ist es eine Aufgabe der vorliegenden Erfindung, eine Maschine zur extrakorporalen Blutbehandlung bereit zu stellen, die einen höheren Entkeimungsgrad auf Seiten des (maschinenseitigen) Flüssigkeitskreislaufs erreicht. Ein bevorzugtes Ziel ist es ferner, dass die Maschine effizienter und schneller arbeitet. Ein weiteres bevorzugtes Ziel ist es, den Energieverbrauch der Maschine zu verringern.

Die vorstehende Aufgabe sowie die weiteren Ziele der Erfindung werden durch eine gattungsgemäße Maschine mit den Merkmalen des Anspruchs 1 gelöst. Vorteilhafte Ausgestaltungen der Erfindung sind Gegenstand der Unteransprüche.

Der Kern der Erfindung besteht demzufolge darin, das Zirkulationsventil zum Umschalten zwischen dem Blutreinigungsmodus und dem Desinfektionsmodus stromab zum Wärmetauscher anzuordnen. Dadurch wird der Wärmetauscher auch in den Desinfektionskreislauf zwischengeschaltet, wodurch er sich im Verlauf des Desinfektionszyklus auf nahezu die Temperatur der Desinfektionsflüssigkeit aufheizt. Diese ist so hoch, dass Keime auf der Zulauf - Seite (durch die Wirkung des Desinfektionsmittels selbst) wie auch auf der Abfluss - Seite des Wärmetauschers (Aufgrund der hohen Temperatur) abgetötet werden können.

Erfindungsgemäß ist eine Wärmetauscher - Bypassleitung vorgesehen, die während des Desinfektionszyklus und/oder während der Spül- und Abkühlphase den Wärmetauscher auf der Zulaufseite brückt, sodass dieser das zugeführte Reinigungs - Abkühlwasser zur Spülen des Flüssigkeitskreislaufs während/am Ende des Desinfektionszyklus nicht aufheizt. Dadurch kann die Abkühlung des Flüssigkeitskreislaufs am Ende des Desinfektionszyklus beschleunigt werden.

Vorteilhaft wäre die Verwendung eines Wärmetauschers mit einer Wärmeübertragungsfläche von ca. 0.4-0.6 m² und vorzugsweise 0.5 m². Dadurch wird zum Einen eine gute Wärmeenergierückgewinnung im Blutreinigungsmodus und eine ausreichende Aufheizung der Zulaufseite des Wärmetauschers im Desinfektionsmodus zur Keimreduktion erzielt.

Vorteilhaft wäre es auch, wenn der Wärmetauscher einen Strömungswiderstand bildet, der größer ist als seine Ungehungsleitung. Damit ist es unnötig, dem Wärmetauscher ein Sperrventil vorzuschalten, sodass sich die Zahl der zu schaltenden Hydraulikelemente klein halten lässt.

Durch die Erfindung können die folgenden Vorteile gegenüber dem Stand der Technik erzielt werden:
- Die erfindungsgemäße Anordnung ermöglicht eine adäquate Desinfektion der Eingangsseite (Zulaufseite) des Wärmetauschers infolge Hitze durch Wärmeübertragung von der Ausgangsseite (Abflussseite).
- Die erfindungsgemäße Anordnung ermöglicht eine adäquate Desinfektion der Ausgangsseite (Abflussseite) infolge Hitze und Desinfektionswirkung des Desinfektionsmittels.
- Die Energieeffizienz der Maschine wird erhöht, da der abgelaufenen Dialysierflüssigkeit ein Großteil der beim Aufheizen zugeführten Energie entzogen werden kann. Die wird möglich durch Verwendung hocheffizienter, großflächiger Wärmetauscher ohne Rücksicht auf eine (nicht mehr vorhandene) Verkeimung der Zulaufseite.
- Die neue erfindungsgemäße Konstruktion des Flüssigkeitskreislaufs (mit Temperaturschalter) ist ohne Änderungen (beispielsweise der Maschinensteuerung) in jedem Blutreinigungssystem der einschlägigen Gattung einsetzbar.
- Die Maschine verbraucht weniger Energie und ist daher gegenüber bekannten Lösungen kostengünstig und effektiv.

Die Erfindung wird nachstehend anhand eines bevorzugten Ausführungsbeispiels unter Bezugnahme auf die begleitenden Figuren näher erläutert. Es zeigen:
Fig. 1 den Flüssigkeitskreislauf einer Maschine zur extrakorporalen Blutbehandlung gemäß dem Stand der Technik,
Fig. 2 den Flüssigkeitskreislauf einer Maschine zur extrakorporalen Blutbehandlung gemäß einem bevorzugten Ausführungsbeispiel der vorliegenden Erfindung und
Fig. 3 die prinzipielle Darstellung eines Wärmetauschers, wie er für den Flüssigkeitskreislauf gemäß der Fig. 2 besonders geeignet ist.

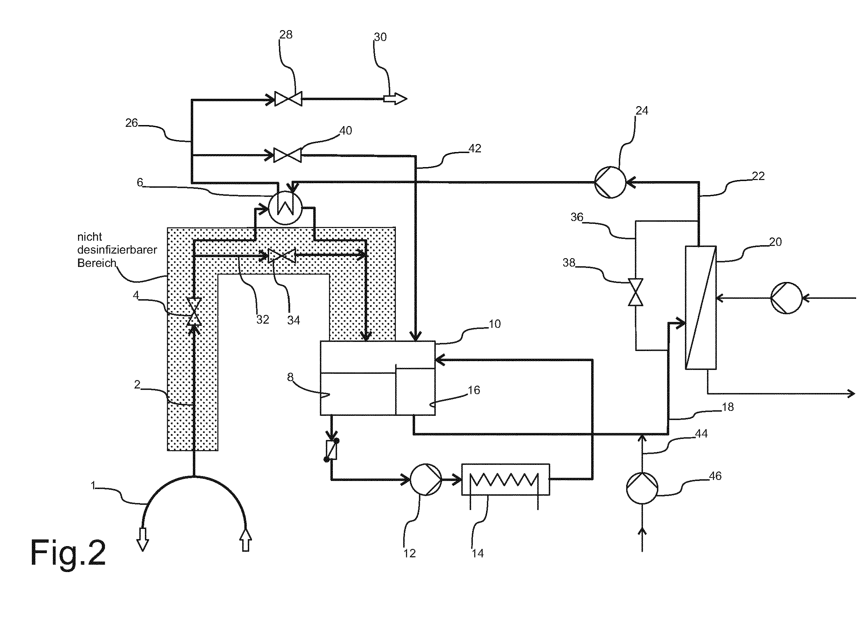

Bei dem in Fig. 2 dargestellten Fluidkreis handelt es sich bevorzugt um einen Reinigungsflüssigkeits- bzw. Dialysierflüssigkeitskreislauf einer Blutreinigungs- bzw. Dialysemaschine. Es kann aber auch ein Flluidkreis für andere extrakorporale Blutreinigungverfahren wie beispielsweise der Reduzierung von Cholesterin im Blut oder dergleichen sein.

Im vorliegenden Ausführungsbeispiel einer Dialysemaschine ist der maschinenseitige Fluidkreis (Dialysierflüssigkeitskreislauf) an eine stationäre Osmose - oder Wasser - Ringleitung 1 eines Dialysezentrums angeschlossen bzw. anschließbar, wobei das Wasser für eine Dialysebehandlung geeignet sein sollte. Über eine Wasser - Zulaufleitung 2, in die ein Zulaufventil (Sperrventil) 4 zwischengeschaltet ist, wird das (dialysegeeignete) Wasser in die Zulaufseite eines Wärmetauschers 6 für dessen Erwärmung geleitet, von wo es in nunmehr (vor-) aufgewärmtem Zustand in einen ersten Tank 8 einer Wasser - Aufbereitungsanlage 10 gelangt. Von dort wird das Wasser unter einem Entgasungsprozess von einer Ansaugpumpe 12 in einen Erhitzer 14 gefördert, in der das Wasser auf eine Temperatur von ca. 37°C erhitzt wird, bevor es dann in einen zweiten Tank 16 der Wasser - Aufbereitungsanlage 10 geleitet wird. Von hieraus wird das zwischengespeicherte, ca. 37°C warme (Dialyse geeignete) Wasser über eine erste Zuführleitung 18 unter Zumischung eines Dialysekonzentrats (nicht weiter dargestellt) und damit nunmehr als frische / unverbrauchte Dialysierflüssigkeit einem Dialysator 20 zugeführt, um dort über eine Dialysatormembran (nicht gezeigt) nach einem bekannten Dialyseverfahren Schadstoffe aus dem Patientenblut aufzunehmen. Die danach als verbraucht geltende Dialysierflüssigkeit wird dann über eine zweite Zuführleitung 22 mit einer zwischengeschalteten Flusspumpe 24 zur Abflussseite des Wärmetauschers 6 geleitet, in welchem es seine Wärme an das erneut eingeleitete Wasser (z.B. Osmose - Wasser) abgibt.

Schließlich gelangt die verbrauchte sowie abgekühlte Dialysierflüssigkeit über eine Abflussleitung 26 und zwischengeschaltetem Abflussventil 28 in einen Abfluss 30 für dessen Entsorgung.

Wie aus der Fig. 2 ferner zu entnehmen ist zweigt stromab zum Zulaufventil 4 eine Umgehungsleitung 32 von der Zulaufleitung 2 ab, welche den Ein- und Ausgang der Zulaufseite des Wärmetauschers 6 verbindet und damit den Wärmetauscher 6 fluiddynamisch kurzschließt. In die Umgehungsleitung 32 ist zusätzlich ein Wärmetauscher - Bypassventil (Sperrventil) 34 zwischengeschaltet. Eine ähnliche Umgehungsleitung 36 findet sich am Dialysator 20, welche die erste und zweite Zuführleitung 18, 22 unter Kurzschließen des Dialysators 20 verbindet und in der ebenfalls ein Dialysator - Bypassventil (Sperrventil) 38 zwischengeschaltet ist.

Parallel zum Abflussventil 28 ist ein Zirkulationsventil 40 stromab an die Abflussseite des Wärmetauschers 6 angeschlossen. Konkret zweigt von der Abflussleitung 26 zwischen Wärmetauscher 6 und Abflussventil 28 eine Zirkulationsleitung 42 ab, in die das Zirkulationsventil (Sperrventil) 40 zwischengeschaltet ist und welche in die Wasser - Aufbereitungsanlage 10, vorzugsweise den ersten Tank 8 mündet.

Schließlich ist eine Desinfektionsleitung 44 an die erste Zuführleitung 18 zwischen Dialysator 20 und zweitem Tank 16 der Wasser - Aufbereitungsanlage 10 angeschlossen, in die eine Förderpumpe 46 zwischengeschaltet ist, welche im Betriebszustand Desinfektionsmittel aus einem nicht weiter dargestellten Desinfektionsmittelbehälter in die erste Zuführleitung 18 pumpt und im Stillstand (außer Betriebszustand) die Desinfektionsleitung 44 fluiddicht sperrt.

Die Funktion der Blutreinigungsmaschine gemäß der Fig. 2 lässt sich wie Folgt beschreiben:
In einem Blutreinigungsmodus (offener Fluidkreis) wird das Wasser aus der Wasser- bzw. Ringleitung 1 in den Wärmetauscher 6 gefördert und dort vorgewärmt. Dann gelangt es in den ersten Tank 8 der Wasser - Aufbereitungsanlage 10. Demzufolge ist das Zulaufventil 4 geöffnet und das Wärmetauscher - Bypassventil 34 geschlossen.

Nachdem das Wasser den Erhitzer 14 (vorzugsweise eine elektrische Heizung) durchlaufen hat und mit einer Temperatur von ca. 37°C in dem zweiten Tank 16 der Wasser - Aufbereitungsanlage 10 zwischengespeichert wurde, wird diesem ein Dialysekonzentrat über eine weitere nicht gezeigte Zweigleitung zugemischt, wodurch sich die eigentliche Dialysierflüssigkeit ausbildet. Diese gelangt über die erste Zuführleitung 18 in den Dialysator 20 und wird von dort als verbrauchte Dialysierflüssigkeit durch die Flusspumpe 24 in der zweiten Zuführleitung 22 zur Abflussseite des Wärmetauschers 6 gepumpt. Nach dem Wärmeübergang auf das nachgeförderte frische Wasser gelangt die abgekühlte, verbrauchte Dialysierflüssigkeit über das Abflussventil 28 in den Ablauf 30. In diesem Fall ist also das Abflussventil 28 geöffnet, wohingegen das Zirkulationsventil 40 und das Dialysator - Bypassventil 38 geschlossen sind.

In einem Desinfektionsmodus (geschlossener Fluidkreis) werden zunächst das Zirkulationsventil 40, das Wärmetauscher - Bypassventil 34 und das Dialysator - Bypassventil 38 geöffnet und das Abflussventil 28 geschlossen. Gleichzeitig wird die Desinfektionspumpe 46 in Betrieb genommen. Demzufolge gelangt frisches Wasser unter Umgehung des Wärmetauschers 6 in die Wasser - Aufbereitungsanlage 10 (erster Tank 8) und von dort in den Erhitzer 14, der das Wasser auf eine Temperatur von ca. 85°C oder mehr erhitzt. Nach erneutem Durchlaufen der Wasser - Aufbereitungsanlage 10 (zweiter Tank 16) wird das heiße Wasser in der ersten Zuführleitung 18 mit Desinfektionsmittel versetzt und durchströmt so die Dialysator - Umgehungsleitung 36, die nachgeschaltete zweite Zuführleitung 22 und die Abflussseite des Wärmetauschers 6. Darauf hin wird das Wasser - Desinfektionsgemisch über die Zirkulationsleitung 42 und das geöffnete Zirkulationsventil 40 in die Wasser - Aufbereitungsanlage 10 zurück geleitet, worauf der vorstehend beschriebene Kreislauf erneut beginnt.

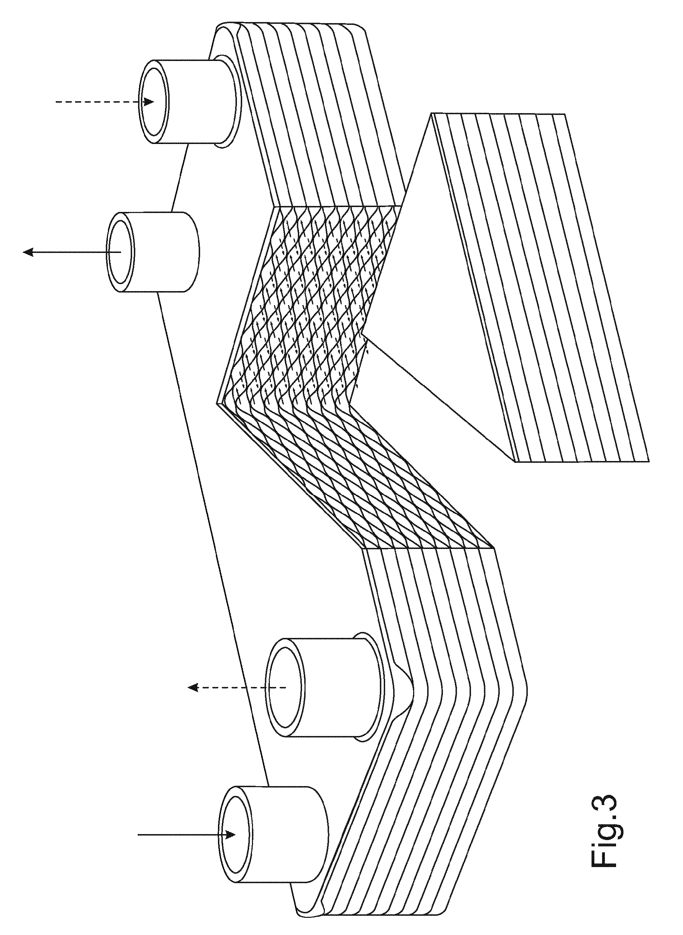

Da im Desinfektionsmodus die Zulaufseite des Wärmetauschers 6 gebrückt ist, also nicht ständig von nachlaufendem Wasser aus der Ringleitung 1 gekühlt wird, heizt sich der Wärmetauscher 6 durch das heiße Desinfektionsmittel auf seiner Abflussseite insgesamt (schnell) auf, sodass auch seine Zulaufseite hitzebedingt desinfiziert / entkeimt wird. Diese Entkeimungswirkung wird um so größer, je größer die Wärmeübertragungsfläche des Wärmetauschers 6 ist. Es hat sich dabei als besonders vorteilhaft gezeigt, wenn der Wärmetauscher 6 gemäß der Fig. 3 vorzugsweise nach dem Gegenstromprinzip in den Fluidkreis zwischengeschaltet ist. Der Wärmetauscher 6 ist gemäß dem vorliegenden bevorzugten Ausführungsbeispiel der Erfindung aus einem Edelstahl gefertigt und hat weiter vorzugsweise eine Wärmeaustauschfläche von zwischen 0.4 m² und 0.6 m² insbesondere 0.5 m². Aufgrund dieser großen Fläche und des im Wärmetauscher 6 vorhandenen Gegenstromprinzips ist ein hoher Wirkungsgrad der Wärmeübertragung gewährleistet. Dadurch ist es möglich, eine maximale Energierückgewinnung im Blutreinigungsmodus zu erzielen und gleichzeitig eine optimale Entkeimung im Desinfektionsmodus zu erreichen.

Gegen Ende des Desinfektionsmodus wird die Desinfektionspumpe 46 abgeschaltet und das Zirkulationsventil 40 geschlossen. Anschließend wird das Abflussventil 28 geöffnet. In dieser Schaltstellung wird frisches Wasser unter Umgehung des Wärmetauschers 6 und des Dialysators 22 im offenen Kreislauf durch den Fluidkreis gepumpt, ohne dass der Erhitzer 14 betätigt wird. D.h., der Fluidkreis wird während dieses Durchspülprozesses maximal gekühlt und auf die für den Blutreinigungsmodus optimale Temperatur zurückgeführt. Nach Abkühlen des Fluidkreises werden die beiden Bypassventile 34, 38 geschlossen und der Heizer 14 auf 37°C eingestellt.

Wie aus der vorstehenden Funktionsbeschreibung zu ersehen ist, ist die Menge an zulaufendem frischem Wasser und ablaufender verbrauchter Dialysierflüssigkeit im Wesentlichen gleich. Bei Verwendung des vorstehenden hocheffizienten Wärmetauschers 6 wird der Großteil der Wärme auf das zulaufende Wasser übertragen. In der Desinfektionsphase kann in einer ersten Ausführung das Wärmetauscher - Bypassventil 34 durch einen Temperaturschalter oder in einer zweiten Ausführung softwaretechnisch (steuerungstechnisch) geschaltet werden, um das zulaufende Wasser am Wärmetauscher 6 vorbei zu führen. Der Temperaturschalter ist vorzugsweise direkt am Wärmetauscher 6 oder direkt im Desinfektionskreislauf eingesetzt / angebracht.

Der Temperaturschalter kann so ausgelegt sein, dass das Wärmetauscher - Bypassventil 34 bei Dialysebetrieb sicher geschlossen ist (z.B. < 45°C) und dass das Wärmetauscher - Bypassventil 34 bei Desinfektion und während des Ausspülens sicher offen ist (z.B. > 45°C). Das frische Wasser verweilt somit auf der Zulaufseite des Wärmetauschers 6 und wird durch Konduktion passiv erhitzt. Das führt wiederum zu einer Keimreduktion, die einer adäquaten Desinfektion von 10⁻⁵ entspricht. Des Weiteren wird verhindert, dass sich die Abkühlphase der Maschine verlängert, da das zulaufende frische Wasser nicht durch die ablaufende verbrauchte Flüssigkeit im Wärmetauscher 6 erhitzt wird.

Zusammenfassend wird eine Blutbehandlungsmaschine bzw. eine Maschine zur extrakorporalen Blutbehandlung offenbart mit einem maschinenseitigen Fluidkreislauf unter anderem aufweisend:
ein Wärmetauscher, der an seiner Zulaufseite frisch zugeführtes, kaltes Reinigungsfluid oder für die Blutreinigung geeignetes Wasser aufwärmt, wofür an seiner gegenüberliegenden Abflussseite verbrauchtes, noch warmes Reinigungsfluid für einen Wärmeaustausch entlang strömt und
ein Zirkulationsventil zum Umschalten zwischen einem Blutreinigungsmodus, in welchem das verbrauchte Reinigungsfluid in offenem Fluidkreis durch die Abflusseite des Wärmetauschers hindurch in einen Ablauf entsorgt wird und einem Desinfektionsmodus, in welchem heißes Desinfektionsmittel in geschlossenem Fluidkreis zirkuliert. Erfindungsgemäß ist das Zirkulationsventil stromab zum Wärmetauscher bezüglich dessen Abflussseite nachgeschaltet.

## Patentansprüche

1. Maschine zur extrakorporalen Blutbehandlung mit einem maschinenseitigen Fluidkreislauf unter anderem aufweisend:
- ein Wärmetauscher (6), der an seiner Zulaufseite frisch zugeführtes, kaltes Reinigungsfluid oder Wasser aufwärmt, wofür an seiner gegenüberliegenden Abflussseite verbrauchtes, noch warmes Reinigungsfluid oder Dialysierflüssigkeit für einen Wärmeaustausch entlang strömt
- eine Wasser-Aufbereitungsanlage (10) mit einem ersten Tank (8), in den das aufgewärmte frische Reinigungsfluid oder Wasser von dem Wärmetauscher (6) aus gelangt, und
- ein Zirkulationsventil (40) zum Umschalten zwischen einem Blutreinigungsmodus, in welchem das verbrauchte Reinigungsfluid in offenem Fluidkreis durch die Abflussseite des Wärmetauschers (6) hindurch über eine Abflussleitung (26) in einen Ablauf (30) entsorgt wird und einem Desinfektionsmodus, in welchem heißes Desinfektionsmittel zumindest zeitweise in geschlossenem Fluidkreis zirkuliert wird, indem das heiße Desinfektionsmittel über eine Zirkulationsleitung (42), in welcher das Zirkulationsventil (40) zwischengeschaltet ist, in die Wasser-Aufbereitungsanlage (10) zurück geleitet wird, und wobei das Zirkulationsventil (40) stromab zu dem Wärmetauscher (6) auf dessen Abflussseite nachgeschaltet ist und die Zirkulationsleitung (42) von der Abflussleitung (26) abzweigt, **dadurch gekennzeichnet, dass** die Zirkulationsleitung (42) in den ersten Tank (8) der Wasser-Aufbereitungsanlage (10) mündet.

2. Maschine nach Anspruch 1, **dadurch gekennzeichnet, dass** bei Umschalten des Zirkulationsventils (40) in den Desinfektionsmodus der Wärmetauscher (6) an seiner Abflussseite in den zumindest zeitweise geschlossenen Kreis eingebunden ist.

3. Maschine nach Anspruch 1, **gekennzeichnet durch** eine Wärmetauscher - Umgehungsleitung (32), die angepasst und dafür vorgesehen ist zur zumindest zeitweisen Überbrückung der Zulaufseite des Wärmetauschers (6) im Desinfektionsmodus **durch** den Wärmetauscher (6) kurzschließende Verbinden des Ein- und Ausgangs der Zulaufseite des Wärmetauschers (6).

4. Maschine nach Anspruch 3, **dadurch gekennzeichnet, dass** in der Umgehungsleitung (32) ein Sperrventil (34) zwischengeschaltet ist, um die Umgehungsleitung (32) im Desinfektionsmodus wahlweise zu öffnen und im Blutreinigungsmodus zu schließen.

5. Maschine nach einem der vorstehenden Ansprüche, **gekennzeichnet durch** ein Abflussventil (28), das parallel zum Zirkulationsventil (40) der Abflussseite des Wärmetauschers (6) nachgeschaltet ist, um in Abstimmung zum Zirkulationsventil (40) den Ablauf (30) im Blutreinigungsmodus frei zuschalten und im Desinfektionsmodus zu sperren.

6. Maschine nach Anspruch 4, **dadurch gekennzeichnet, dass** der Wärmetauscher (6) aus einem nichtrostenden Stahl insbesondere Edelstahl gefertigt ist und eine Wärmeaustauschfläche zwischen 0.4 m² und 0.6 m² und vorzugsweise von 0.5 m² hat.

7. Maschine nach Anspruch 6, **dadurch gekennzeichnet, dass** der Wärmetauscher (6) im Gegenstromprinzip betrieben ist.

8. Maschine nach einem der Ansprüche 6 und 7, **dadurch gekennzeichnet, dass** der Wärmetauscher (6) einen Strömungswiderstand bildet, der größer ist als der Strömungswiderstand der Wärmetauscher - Umgehungsleitung (32) mit geöffnetem Sperrventil (34).

## Claims

1. A machine for extracorporeal blood treatment comprising a machine-side fluid circulation, inter alia comprising:
- a heat exchanger (6) for heating cold purification fluid or water freshly supplied on its intake side, for which purpose used still warm purification fluid or dialysis fluid flows past its opposite discharge side for heat exchange,
- a water processing unit (10) having a first tank (8) into which the heated, fresh purifying fluid or water is conveyed from the heat exchanger (6),
a circulation valve (40) for changing over between a blood purification mode in which the used purification fluid is disposed of in an open fluid circuit through the discharge side of the heat exchanger (6) via a discharge line (26) into a drain (30) and a disinfection mode in which hot disinfectant is circulated at least temporarily in a closed fluid circuit, by returning the hot disinfectant to the water processing unit (10) via a circulation line (42) in which the circulation valve (40) is interconnected, and wherein the circulation valve (40) is connected downstream of the heat exchanger (6) on the discharge side thereof, and the circulation line (42) branches off of the discharge line (26), **characterized in that** circulation line (42) opens into the first tank (8) of the water processing unit (10).

2. The machine according to claim 1, **characterized in that** when switching the circulation valve (40) into the disinfection mode, the heat exchanger (6) is included in the at least temporarily closed circuit.

3. The machine according to claim 1, **characterized by** a heat exchanger bypass line (32) which is adapted and provided for at least temporarily bridging the intake side of the heat exchanger (6) in the disinfection mode by connecting the input and the output of the intake side of the heat exchanger (6) in a short-circuiting manner.

4. The machine according to claim 3, **characterized in that** in the bypass line (32), a stop valve (34) is interconnected in order to selectively open the bypass line (32) in the disinfection mode and to close it in the blood purification mode.

5. The machine according to one of the preceding claims, **characterized by** a discharge valve (28) connected downstream of the discharge side of the heat exchanger (6) in parallel to the circulation valve (40) so as to open the drain (30) in the blood purification mode and to block it in the disinfection mode in accordance with the circulation valve (40).

6. The machine according to claim 4, **characterized in that** the heat exchanger (6) is made of non-corroding steel, especially stainless steel, and includes a heat exchange area of from 0.4 m² to 0.6 m² and preferably of 0.5 m².

7. The machine according to claim 6, **characterized in that** the heat exchanger (6) is operated based on the counter flow principle.

8. The machine according to one of the claims 6 and 7, **characterized in that** the heat exchanger (6) forms a flow resistance higher than the flow resistance of the heat exchanger bypass line (32) when the stop valve (34) is opened.

## Revendications

1. Machine de traitement extracorporel du sang comprenant un circuit de fluide situé côté machine, présentant entre autres :
- un échangeur de chaleur (6), qui réchauffe, au niveau de son côté d'arrivée, un fluide de nettoyage ou de l'eau fraîchement amené(e), froid(e), un fluide de nettoyage usagé, encore chaud ou un liquide de dialyse circulant à cet effet pour un échange de chaleur le long de son côté de flux sortant opposé,
- une installation de préparation d'eau (10) comprenant un premier réservoir (8), dans lequel le fluide de nettoyage ou l'eau frais/fraîche réchauffé(e) parviennent depuis l'échangeur de chaleur (6), et
- une soupape de circulation (40) servant à commuter entre un mode de nettoyage du sang, dans lequel le fluide de nettoyage usagé dans le circuit de fluide ouvert est éliminé dans une évacuation (30) à travers le côté de flux sortant de l'échangeur de chaleur (6) par l'intermédiaire d'un conduit de flux sortant (26), et un mode de désinfection, dans lequel un agent de désinfection chaud circule au moins par intermittence dans le circuit de fluide fermé l'agent de désinfection chaud étant réacheminé dans l'installation de préparation d'eau (10) par l'intermédiaire d'un conduit de circulation (42), dans lequel la soupape de circulation (40) est intercalée, et dans laquelle la soupape de circulation (40) est montée en aval de l'échangeur de chaleur (6) sur le côté de flux sortant de ce dernier et le conduit de circulation (42) réalise une ramification depuis le conduit de flux sortant (26), **caractérisée en ce que** le conduit de circulation (42) débouche dans le premier réservoir (8) de l'installation de préparation d'eau (10).

2. Machine selon la revendication 1, **caractérisée en ce que** lors de la commutation de la soupape de circulation (40) dans le mode de désinfection, l'échangeur de chaleur (6) est intégré, au niveau de son côté de flux sortant, dans le circuit au moins par intermittence fermé.

3. Machine selon la revendication 1, **caractérisée par** un conduit de contournement d'échangeur de chaleur (32), qui est adapté et prévu afin de ponter au moins par intermittence le côté d'arrivée de l'échangeur de chaleur (6) dans le mode de désinfection par la liaison, court-circuitant l'échangeur de chaleur (6), de l'entrée et de la sortie du côté d'arrivée de l'échangeur de chaleur (6).

4. Machine selon la revendication 3, **caractérisée en ce qu'**une soupape de fermeture (34) est intercalée dans le conduit de contournement (32) afin au choix d'ouvrir dans le mode de désinfection et de fermer dans le mode de nettoyage du sang le conduit de contournement (32).

5. Machine selon l'une quelconque des revendications précédentes, **caractérisée par** une soupape de flux sortant (28), qui est montée en aval de manière parallèle par rapport à la soupape de circulation (40) du côté de flux sortant de l'échangeur de chaleur (6) afin d'activer librement dans le mode de nettoyage du sang et de fermer dans le mode de désinfection l'évacuation (30) en accord avec la soupape de circulation (40).

6. Machine selon la revendication 4, **caractérisée en ce que** l'échangeur de chaleur (6) est confectionné à partir d'un acier ne rouillant pas, en particulier d'un acier inoxydable, et présente une surface d'échange de chaleur comprise entre 0,4 m² et 0,6 m² et de préférence de 0,5 m².

7. Machine selon la revendication 6, **caractérisée en ce que** l'échangeur de chaleur (6) fonctionne selon le principe de circulation à contre-courant.

8. Machine selon l'une quelconque des revendications 6 et 7, **caractérisée en ce que** l'échangeur de chaleur (6) forme une résistance à l'écoulement, qui est plus grande que la résistance à l'écoulement du conduit de contournement d'échangeur de chaleur (32) pourvu d'une soupape de fermeture (34) ouverte.
